(19) Europäisches Patentamt
European Patent Office
Office européen des brevets

(11) Veröffentlichungsnummer: **0 421 219 A2**

# (12) EUROPÄISCHE PATENTANMELDUNG

(21) Anmeldenummer: **90118262.6**

(22) Anmeldetag: **22.09.90**

(51) Int. Cl.[5]: **C07D 501/18**, C07D 205/095

(30) Priorität: **03.10.89 DE 3932905**
**11.10.89 DE 3933934**

(43) Veröffentlichungstag der Anmeldung:
**10.04.91 Patentblatt 91/15**

(84) Benannte Vertragsstaaten:
**AT BE CH DE DK ES FR GB GR IT LI LU NL SE**

(71) Anmelder: **BAYER AG**

**W-5090 Leverkusen 1 Bayerwerk(DE)**

(72) Erfinder: **Lanz, Joachim, Dr.**
**Claudiusweg 9**
**W-5600 Wuppertal 1(DE)**
Erfinder: **Naab, Paul, Dr.**
**Amalienstrasse 29**
**W-5600 Wuppertal 2(DE)**
Erfinder: **Rosentreter, Ulrich, Dr.**
**Kondorweg 23**
**W-5600 Wuppertal 1(DE)**

## (54) Verfahren zur Herstellung von 7-Amino-3-[(Z)-1-propen-1-yl]-3-cephem-4-carbonsäure.

(57) Die Erfindung betrifft ein Verfahren zur Herstellung von 7-Amino-3-[(Z)-1-propen-1-yl]-3-cephem-4-carbonsäure sowie Verfahren zur Herstellung von Zwischenprodukten, die zu dieser Verbindung führen.

EP 0 421 219 A2

## VERFAHREN ZUR HERSTELLUNG VON 7-AMINO-3-[(Z)-1-PROPEN-1-YL]-3-CEPHEM-4-CARBONSÄURE

Die Erfindung betrifft ein Verfahren zur Herstellung von 7-Amino-3-[(Z)-1-propen-1-yl]-3-cephem-4-carbonsäure, die im folgenden auch als 7-APCA oder als Verbindung (I) bezeichnet wird.

7-APCA entspricht der Strukturformel

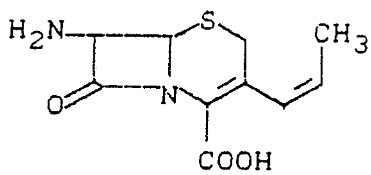

(I)

und ist ein wichtiges Synthesezwischenprodukt für die Herstellung von cephalosporinartigen Antibiotika. Beispielsweise kann 7-APCA in Verbindungen umgewandelt werden wie sie in den EP-PSen 88 107 679.8 und 88 107 686.3 beschrieben sind.

Diese Erfindung beschreibt ein vereinfachtes Verfahren zur Herstellung der Verbindung (I). Dabei werden Verbindungen der allgemeinen Formel (II) in einem einzigen Syntheseschritt in (I) umgewandelt nach dem Schema

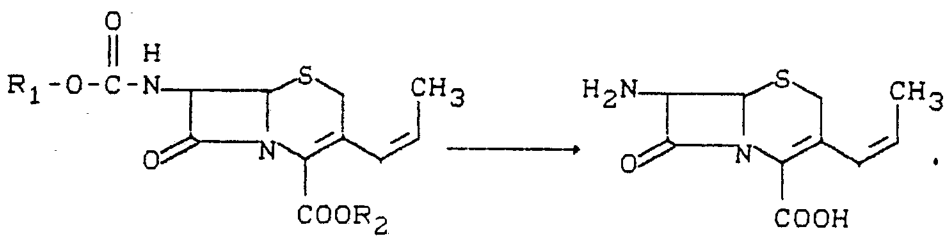

(II) (I)

In der allgemeinen Formel (I) stehen $R_1$ und $R_2$ für Alkyl- und Aralkylreste und können gleich oder verschieden sein.

Die bisherigen Verfahren zur Herstellung von (I) (siehe die DE 3 613 365 A1) gehen von Verbindungen wie solchen der Formel (III) aus, deren Umwandlung in (I) in zwei separaten Schritten erfolgt.

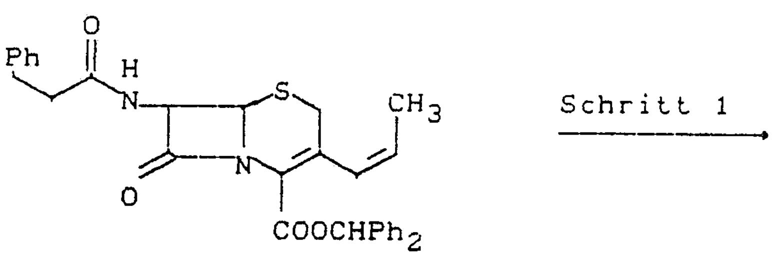


(III)

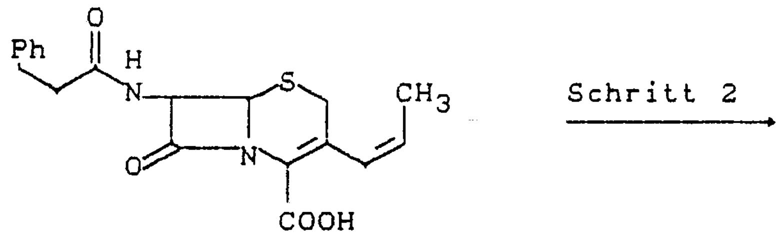


(IV)

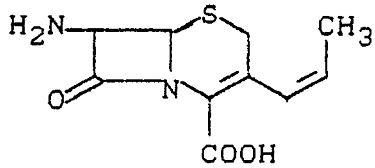

(I)

Dabei wird die Verbindung der Formel (III) im ersten Schritt durch Behandeln mit einer starken Säure wie Trifluoressigsäure in die Verbindung (IV) überführt. Die isolierte Verbindung (IV) wird dann im zweiten Schritt durch eine enzymatische Reaktion in die Verbindung (I) umgewandelt.

Ein anderes, ebenfalls zweistufiges Verfahren zur Herstellung der Verbindung(I) geht ebenfalls von Verbindungen wie solchen der Formel (III) aus.

Hier wird im ersten Schritt durch Behandeln mit z.B. Phosphorpentachlorid die Verbindung (V) hergestellt und isoliert. Verbindung (V) kann dann durch Behandeln mit starken Säuren wie Trifluoressigsäure in die Verbindung (I) überführt werden.

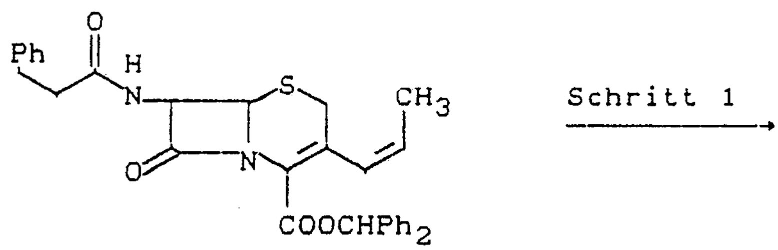


(III)

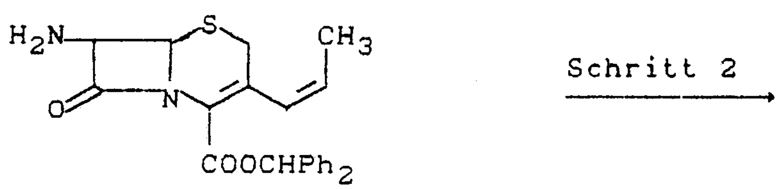


(V)

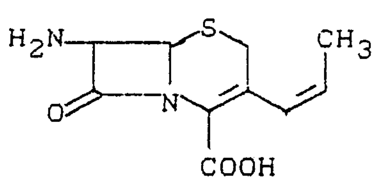

(I)

Bei dem in der vorliegenden Erfindung beschriebenen Verfahren werden die Verbindungen der allgemeinen Formel (II) jedoch in einem einzigen Syntheseschritt ohne Isolierung eventuell auftretender Zwischenprodukte in die Verbindung (I) umgewandelt.

Diese Umwandlung geschieht durch Behandlung der Verbindung (II) mit starken Protonsäuren oder durch Behandlung mit Lewissäuren.

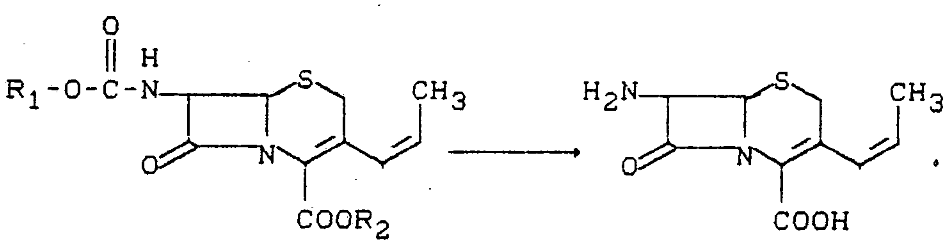

(II) (I)

$R_1$ und $R_2$ stehen für geradkettiges oder verzweigtes $C_1$-$C_{10}$, bevorzugt $C_4$-$C_8$-Alkyl oder $C_6$-$C_{12}$-Aryl bzw. $C_6$-$C_{12}$-Ar-$C_1$-$C_4$-Alkylreste.

Bevorzugte Beispiele für die durch $R_1$ und $R_2$ repräsentierten Gruppen sind tert.-Butyl-, 1,1-Dimethylpropyl-, 1,1-Diethylpropyl-, 1,1-Dimethylbutyl-, 1,1,2,2-Tetramethylpropyl-, Benzyl-, 4-Methylbenzyl-, 4-Methoxybenzyl-, 2,4-Dimethylbenzyl-, 3,4-Dimethoxybenzyl-, 3,4,5-Trimethoxybenzyl-, Diphenylmethyl-, Dimethylphenylmethyl-, Di(4-Methylphenyl)methylgruppen. Dabei können $R_1$ und $R_2$ gleich oder verschieden sein.

Beispiele für starke Protonsäuren sind niedere Alkylcarbonsäuren, Ameisensäure, niedere halogenierte Alkylcarbonsäuren,Arylcarbonsäuren, niedere Alkylsulfonsäuren, niedere halogenierte Alkylsulfonsäuren und Arylsulfonsäuren. Bevorzugte Beispiele sind Essigsäure, Ameisensäure, Trifluoressigsäure, Trichloressigsäure, 4-Nitrobenzoesäure, 2,4-Dinitrobenzosäure, Methansulfonsäure, Trifluormethansulfonsäure, Toluolsulfonsäure, 4-Nitrobenzolsulfonsäure.

Beispiele für Lewissäuren sind Aluminiumhalogenide, Borhalogenide, Titanhalogenide, Zinnhalogenide, Silylhalogenide, Trialkylsilylhalogenide.

Bevorzugte Beispiele sind Aluminiumtrichlorid, Bortrifluorid, Bortrichlorid, Bortribromid, Titantetrachlorid, Zinntetrachlorid, Siliciumtetrachlorid, Trimethylsilylchlorid, Trimethylsilylbromid, Trimethylsilyljodid.

Die Säure kann in äquivalenten Mengen oder im großen Überschuß eingesetzt werden. Flüssige Säuren, wie z.B. Trifluoressigsäure, können auch als Lösungsmittel verwendet werden.

Die Reaktion kann auch in Gegenwart von nucleophilen Verbindungen durchgeführt werden. Anwendbare nucleophile Verbindungen sind beispielsweise Arylthiole, Heteroarylthiole, Arylalkylthioether, Trialkylsilane, Triarylsilane, Trialkylzinnhydride. Bevorzugte Beispiele sind Thioanisol, Thiophenol, Mercaptobenzothiazol, Triethylsilan, Triphenylsilan und Tributylzinnhydrid. Diese nucleophilen Verbindungen können in äquivalenten Mengen oder im Überschuß angewendet werden. Besonders bevorzugt verwendet man 1 bis 4 Äquivalente nucleophile Verbindung.

Beispiele für die in dieser Erfindung anwendbaren Lösungsmittel sind halogenierte niedere Kohlenwassrstoffe, niedere Carbonsäuren, niedere halogenierte Carbonsäuren, aromatische Kohlenwasserstoffe, niedere Alkylether, cyc lische Ether; Ester niederer Carbonsäuren, niedere Alkylnitrile. Bevorzugte Beispiele sind Dichlormethan, Chloroform, Ameisensäure, Essigsäure, Benzol, Toluol, Chlorbenzol, Diethylether, Tetrahydrofuran, Dioxan, Essigsäureethylester, Essigsäurebutylester, Acetonitril. Die Reaktionstemperatur liegt zwischen -40° C und +30° C. Die Reaktion kann unter einem Inertgas wie z.B. Stickstoff bei Normaldruck oder bei erhöhtem Druck durchgeführt werden.

In dieser Erfindung wird außerdem ein Verfahren beschrieben, in dem die Verbindungen der allgemeinen Formel (II) aus Verbindungen der allgemeinen Formel (VI) hergestellt werden, wobei

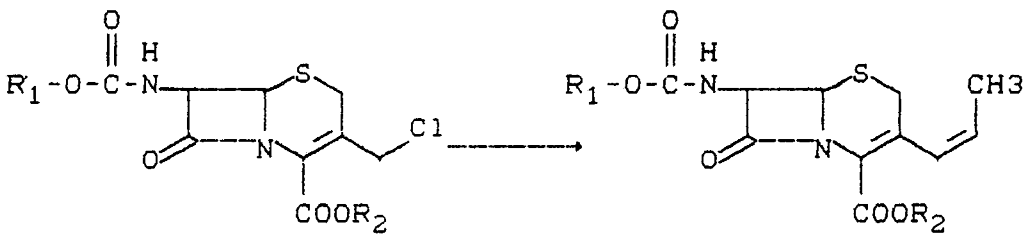

(VI) (II)

$R_1$ und $R_2$ die oben angegebene Bedeutung haben können.

Dabei werden die Verbindungen (VI) zuerst mit Triphenylphosphin und Alkalijodiden und dann mit Acetaldehyd und einer Base behandelt.

Triphenylphosphin wird in 1 bis 2 Äquivalenten zugesetzt. Bevorzugte Alkalijodide sind Natriumjodid und Kaliumjodid. Die Alkalijodide werden mit 0,1 bis 2 Äquivalenten eingesetzt. Der Acetaldehyd wird in 5 bis 50 Äquivalenten zugegeben, vorzugweise werden 10 bis 20 Äquivalente eingesetzt.

In diesem Verfahren anwendbare Basen sind Alkalihydroxide, Erdalkalihydroxide, Alkalicarbonate, Alkalihydrogencarbonate, Alkaliacetate. Bevorzugte Beispiele sind Natriumhydroxid, Kaliumhydroxid, Calciumhydroxid, Natriumcarbonat, Kaliumcarbonat, Natriumhydrogencarbonat und Natriumacetat.

Für dieses Verfahren geeignete Lösungsmittel sind halogenierte, niedere Kohlenwasserstoffe, aromatische Kohlenwasserstoffe, niedere Alkylether, cyclische Ether, Ester niederer Carbonsäuren, niedere Alkylketone, niedere Alkohole, Carbonsäureamide und Alkylnitrile.

Besonders bevorzugte Beispiele sind Dichlormethan, Chloroform, 1,2-Dichlorethan, Toluol, Chlorbenzol, Diethylether, Diisopropylether, tert.-Butylmethylether, Tetrahydrofuran, Dioxan, Essigsäureethylester, Essigsäurebutylester, Methanol, Ethanol, Isopropanol, n-Butanol, Acetamid, Dimethylformamid, Acetonitril. Auch Mischungen dieser Lösungsmittel untereinander oder mit Wasser können zur Anwendung kommen.

Die Reaktionstemperatur kann zwischen -5° C und +70° C liegen.

Die Reaktion kann bei Normaldruck, aber auch unter erhöhtem Druck durchgeführt werden.

Außerdem betrifft die Erfindung ein Verfahren zur Herstellung der Verbindungen der allgemeinen Formel (VI) aus den Verbindungen der allgemeinen Formel (VII), wobei $R_1$ und $R_2$ die oben angegebenen Bedeutungen haben können.

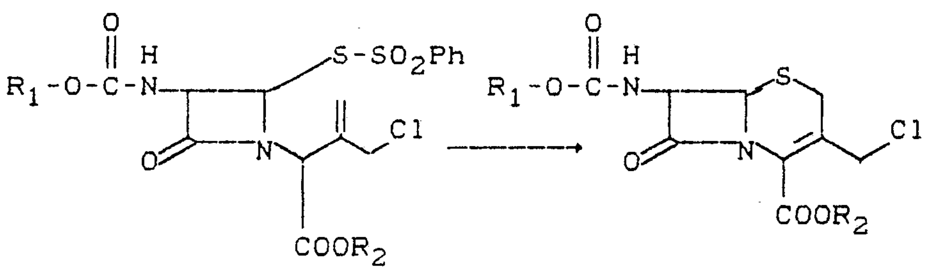

(VII) (VI)

Dabei werden die Verbindungen (VII) in einem polaren Lösungsmittel bei tiefer Temperatur mit einer Base behandelt.

Für dieses Verfahren geeignete polare Lösungsmittel sind Carbonsäureamide, cyclische Harnstoffderivate. Besonders bevorzugte Beispiele sind Dimethylformamid, Dimethylacetamid, Acetamid, 1,3-Dimethyl-2-imidazolidinon, 1,3-Dimethyltetrahydro-2(1H)-pyrimidinon oder Gemische dieser Lösungsmittel. Auch Mischungen dieser Lösungsmittel mit Wasser sind möglich.

Für dieses Verfahren geeignete Basen sind Ammoniak, Alkylamine, Alkalihydroxide, Alkalicarbonate, Alkalihydrogencarbonate, Alkalifluoride. Besonders bevorzugt sind Ammoniak, Methylamin, Ethylamin, Triethylamin, Diisopropylamin, Natriumhydroxid, Kaliumhydroxid, Natriumcarbonat, Kaliumcarbonat, Natriumhydrogencarbonat.

Diese Basen können in äquivalenten Mengen, aber auch im Überschuß eingesetzt werden. Besonders bevorzugt verwendet man 1 bis 4 Äquivalente Base.

Die Reaktionstemperatur kann zwischen -70° C und 0° C liegen, bevorzugterweise arbeitet man zwischen -50° C und -10° C.

Weiterhin wird ein Verfahren zur Herstellung der Verbindungen (VII) aus den Verbindungen der allgemeinen Formel (VIII) beschrieben, wobei $R_1$ und $R_2$ die oben angegebenen Bedeutungen haben.

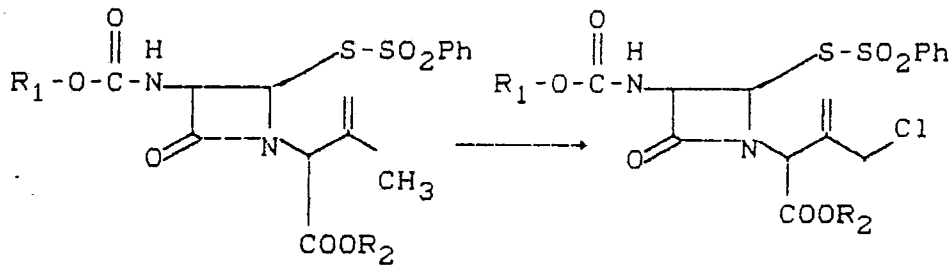

(VIII) (VII)

Bei diesem Verfahren werden die Verbindungen (VIII) in einem organischen Lösungsmittel mit einem Chlorierungsmittel behandelt.

Für dieses Verfahren geeignete Lösungsmittel sind halogenierte, niedere Kohlenwasserstoffe, aromatische Kohlenwasserstoffe, niedere Alkylether, cyclische Ether, Ester niederer Carbonsäuren, niedere Carbonsäureamide.

Besonders bevorzugte Beispiele sind Dichlormethan, Chloroform, Tetrachlorkohlenstoff, Toluol, Chlorbenzol, Diethylether, tert.-Butylmethylether, Tetrahydrofuran, Dioxan, Essigsäureethylester, Essigsäurebutylester, Dimethylformamid. Diese Lösungsmittel können auch im Gemisch mit Wasser angewendet werden.

Für dieses Verfahren geeignete Chlorierungsmittel sind Chlor, Alkylhypochlorit, hypochlorige Säure, Chlordioxid und Sulfurylchlorid. Besonders bevorzugte Beispiele sind Chlor, tert.-Butylhypochlorit, Chlordioxid, hypochlorige Säure und Sulfurylchlorid.

Das Chlorierungsmittel kann in äquivalenten Mengen oder im Überschuß angewendet werden. Im allgemeinen arbeitet man mit 1 bis 2 Äquivalenten Chlorierungsmittel. Die Reaktionstemperatur kann zwischen -40° C und +40° C liegen, besonders bevorzugt arbeitet man zwischen -30° C und +20° C.

Schließlich wird in dieser Erfindung ein Verfahren zur Herstellung der Verbindungen (VIII) aus Verbindungen mit der allgemeinen Formel (IX) beschrieben,

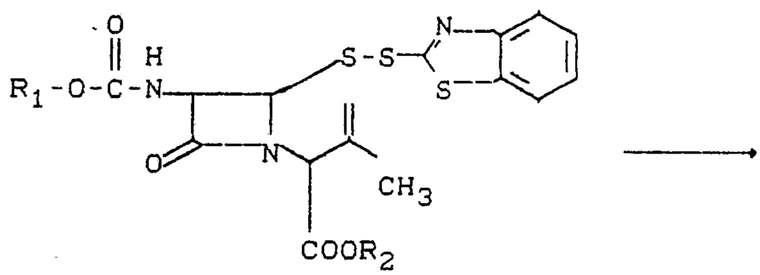

(IX)

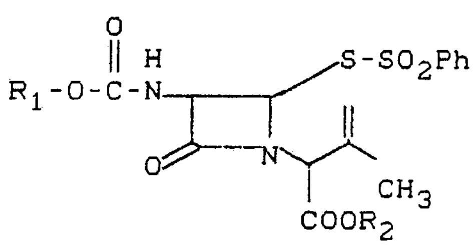

(VIII)

wobei $R_1$ und $R_2$ die oben angegebene Bedeutung haben.

Bei diesem Verfahren werden die Verbindungen (IX) durch Behandeln mit Benzolsulfinsäure, gegebenenfalls in Gegenwart eines Oxidationsmittels in die Verbindungen (VIII) umgewandelt.

Für dieses Verfahren geeignete Lösungsmittel sind niedere Alkylalkohole, aromatische Kohlenwasserstoffe, halogenierte niedere Kohlenwasserstoffe, Ester niederer Carbonsäuren, niedere Alkylether. Besonders bevorzugte Beispiele sind Methanol, Ethanol, Isopropanol, Benzol, Toluol, Xylol, Chlorbenzol, Essigsäureethylester, Essigsäurebutylester, Dichlormethan, Chloroform, Diethylether, tert.-Butylmethylether, Diisopropylether. Es sind auch Mischungen dieser Lösungsmittel untereinander oder mit Wasser möglich.

Die bei diesem Verfahren verwendete Benzolsulfinsäure kann in äquivalenter Menge oder im Überschuß angewendet werden. Besonders bevorzugt wird mit 1 bis 4 Äquivalenten gearbeitet. Die Benzolsulfinsäure kann auch in situ aus Benzolsulfinsäurenatriumsalz durch Zugabe von starken Säuren wie z.B. Salzsäure oder Schwefelsäure erzeugt werden.

Die bei diesem Verfahren gegebenenfalls verwendeten Oxidationsmittel sind z.B. Wasserstoffsuperoxid, Chloramin T, Chloramin B, Chlorlauge, Calciumhypochlorit, Lithiumhypochlorit, Chlorkalk, N-Chlorsuccinimid und Chlor. Besonders bevorzugt ist Wasserstoffsuperoxid als Oxidationsmittel.

Die Reaktionstemperatur kann zwischen 0° C und 30° C liegen.

Bei Verwendung eines Oxidationsmittels können auch Katalysatoren wie Alkalijodide vorteilhaft angewendet werden.

Die Synthese der Verbindungen der allgemeinen Formel (IX) erfolgt analog zu A.I. Scott, R. Shankaranarayan, Tetrahedron Letters 29 , 3179-3182 (1988).

Beispiel 1

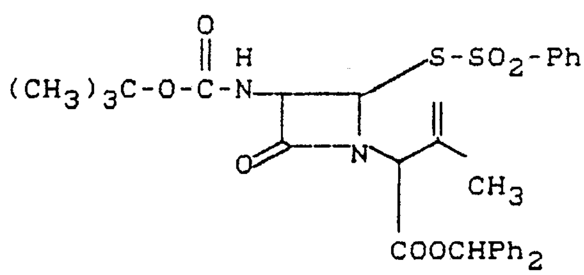

2-[4-(Benzolsulfonylthio)-3-tert.-butyloxycarbonylamido-2-oxo-azetidin-1-yl]-3-methylenbuttersäurediphenylmethylester

A. Synthese des Ausgangsmaterials

A.1.

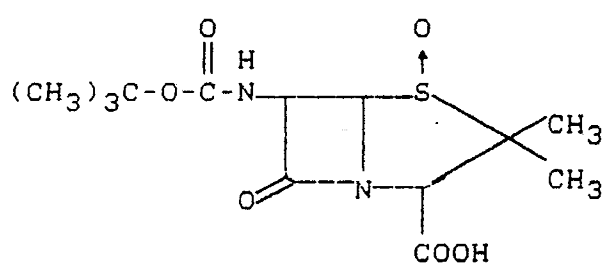

6-tert.-Butyloxycarbonylamido-penicillansäure-1-oxid

216 g (1 Mol) 6-Aminopenicillansäure werden in 2000 ml Ethanol und 200 ml Wasser suspendiert. Dann werden 140 ml (1 Mol) Triethylamin zugegeben, 15 Min. gerührt und zur klaren Lösung 240 g (1,11 Mol) Pyrokohlensäureditert.-butylester zugegeben. Die Reaktionsmischung wird 1 h bei 20° C gerührt, dann am Rotationsverdampfer im Vakuum auf ca. 1000 ml Volumen eingeengt. Der Rückstand wird mit 1500 ml

Wasser versetzt und nach Zugabe von 87 g Natriumhydrogencarbonat mit 500 ml Essigester extrahiert. Die organische Phase wird abgetrennt, die wäßrige Phase wird mit 1500 ml Essigester überschichtet und mit 2n Schwefelsäure auf pH = 3 gebracht. Die organische Phase wird abgetrennt, die wäßrige Phase noch zweimal mit je 500 ml Essigester extrahiert. Die vereinigten organischen Phasen werden im Vakuum eingedampft, der resultierende ölige Rückstand in einer Lösung von 87 g Natriumhydrogencarbonat in 2000 ml Wasser gelöst. Nach Zugabe von 4 g Natriumwolframat-Dihydrat werden bei 15 bis 20° C 110 ml 30 % Wasserstoffsuperoxid innerhalb von 15 Min. zugetropft. Die Reaktionslösung wird 1,5 h bei 15 bis 20° C nachgerührt, dann mit 1000 ml Essigester überschichtet und mit 2n Schwefelsäure auf pH = 3 gebracht. Der dabei ausfallende Niederschlag wird abgesaugt, mit 1000 ml Wasser und wenig Essigester gewaschen und im Vakuum getrocknet.
Ausbeute: 201 g Produkt = 60,5 % der Theorie.
NMR (250 MHZ, $CDCl_3$): 1,2[3]s, 1,4[9]s, 1,6[3]s, 4,35 [1]s, 5,45[1]d J = 5Hz, 5,6[1]dd J = 10Hz J = 5Hz, 6,2[1]d J = 10Hz.
$R_f$-Wert: 0,33 (Mobile Phase: Organische Phase einer kräftig geschüttelten Mischung von 300 ml Butylacetat, 36 ml n-Butanol, 100 ml Eisessig und 60 ml Pufferlösung pH 7).

A.2.

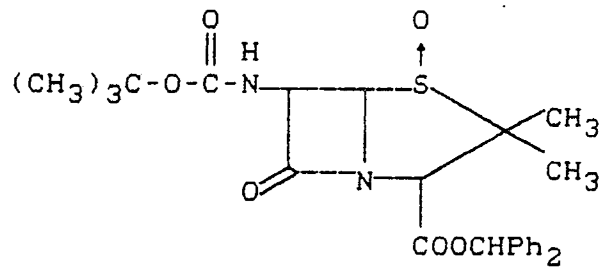

6-tert.-Butyloxycarbonylamido-penicillansäure-diphenylmethylester-1-oxid

132,8 g (0,4 Mol) 6-tert.-Butyloxycarbonylamido-penicillansäure-1-oxid werden in 400 ml Toluol suspendiert und mit 520 ml einer 0,8 molaren Lösung von Diphenyldiazomethan in Toluol versetzt. Die Reaktionslösung wird über Nacht bei 20° C gerührt und dann mit 1000 ml Diisopropylether versetzt. Es wird 1 h bei 10° C gerührt, abgesaugt, mit 500 ml Diisopropylether gewaschen und im Vakuum getrocknet.
Ausbeute: 166,9 g Produkt = 83,7 % der Theorie.
NMR (250 MHZ, $CDCl_3$): 0,9[3]s, 1,45[9]s, 1,7[3]s, 4,75[1]s, 5,0[1]d J = 5Hz, 5,7[1]dd J = 10Hz J = 5Hz, 6,05[1]d J = 10Hz, 7,0[1]s, 7,3-7,45[10]m
$R_f$-Wert: 0,55 (Toluol/Essigester 8:2)

A.3.

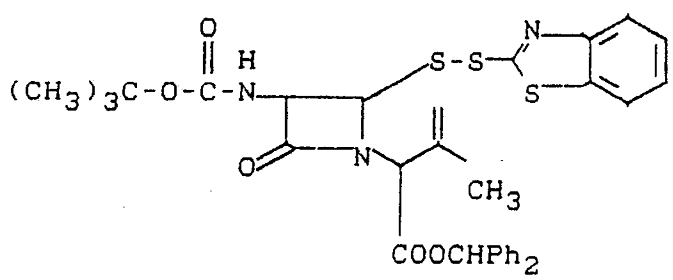

2-[4-(Benzthiazol-2-yldithio)-3-tert.-butyloxycarbonylamido-2-oxo-azetidin-1-yl]-3-methylenbuttersäure-diphenylmethylester

60 g (0,12 Mol) 6-tert.-Butyloxycarbonylamido-penicillansäure-diphenylmethylester-1-oxid werden zusammen mit 20,4 g (0,12 Mol) 2-Mercaptobenzothiazol in 850 ml Toluol 1,5 h am Wasserabscheider unter Rückfluß erhitzt. Nach Abkühlen wird die Reaktionslösung im Vakuum eingedampft. Der ölige Rückstand

wird über Nacht in 400 ml Diisopropylether gerührt. Der entstandene kristalline Niederschlag wird abgesaugt und mit Diisopropylether gewaschen. Dieses Rohprodukt wird in 500 ml Diisopropylether zum Rückfluß erhitzt und mit 60 ml Essigester bei Rückflußtemperatur in Lösung gebracht. Die heiße Lösung wird filtriert und langsam abgekühlt. Das ausgefallene Produkt wird abgesaugt, mit Diisopropylether gewaschen und im Vakuum getrocknet.
Ausbeute: 40,8 g Produkt = 52 % der Theorie.
NMR (250 MHZ, $CDCl_3$): 1,45[9]s, 1,9[3]s, 4,95[1]s, 5,05[1]s, 5,15[1]s, 5,3[1]dd J = 10Hz J = 5Hz, 5,5[1]d J = 5Hz, 5,55[1]d J = 10Hz, 6,9[1]s, 7,2-7,35[11]m, 7,45[1]ddd J = 7Hz J = 7Hz J = 2Hz, 7,75[1]dd J = 7Hz J = 2Hz, 7,9[1]dd J = 7Hz J = 2Hz
$R_f$-Wert: 0,6 (Toluol/Essigester 8:2).

B.

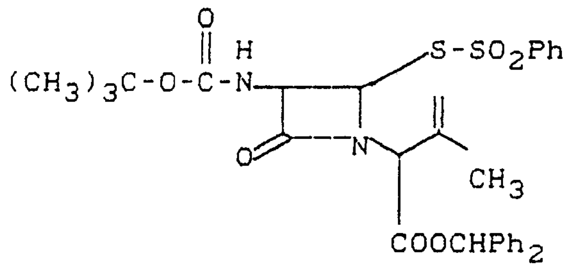

2-[4-(Benzolsulfonylthio)-3-tert.-butyloxycarbonylamido-2-oxo-azetidin-1-yl]-3-methylenbuttersäure-diphenylmethylester

62,5 g (0,1 Mol) 2-[4-Benzthiazol-2-yldithio)-3-tert.-butyloxycarbonylamido-2-oxo-azetidin-1-yl]-3-methylenbuttersäure-diphenylmethylester werden in 250 ml Toluol suspendiert und mit 250 ml gesättigter Kochsalzlösung versetzt. Anschließend werden 32,8 g (0,2 Mol) Benzolsulfinsäure-Natriumsalz und 40 ml 6n Salzsäure zugegeben, 2,5 h bei 20 °C gerührt und der entstandene Nieder schlag abgesaugt. Im Filtrat werden die Phasen getrennt und zur organischen Phase 7 ml 30 % Wasserstoffperoxid gegeben. Die Reaktionsmischung wird über Nacht bei 20 °C gerührt, im Vakuum eingedampft und der Rückstand in 700 ml Diisopropylether ausgerührt. Der entstandene kristalline Niederschlag wird abgesaugt, mit Diisopropylether gewaschen und im Vakuum getrocknet.
Ausbeute: 32 g Produkt = 51,4 % der Theorie.
NMR (250 MHZ, $CDCl_3$): 1,4[9]s, 1,75[3]s, 4,4[1]s, 4,75[1]s, 4,85[1]s, 5,0[2]m, 5,75[1]d J = 5Hz, 6,85[1]s, 7,3-7,6[13]m, 7,8[2]d J = 7Hz.
$R_f$-Wert: 0,5 (Toluol/Essigester 8:2).

Beispiel 2

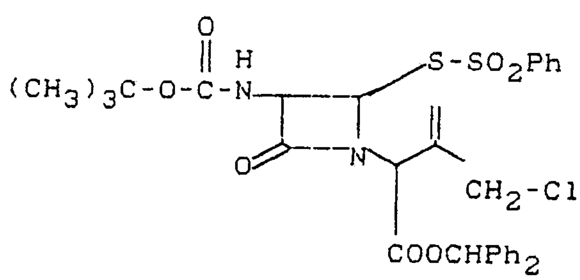

2-[4-(Benzolsulfonylthio)-3-tert.-butyloxocarbonylamido-2-oxo-azetidin-1-yl]-4-chlor-3-methylenbuttersäure-diphenylmethylester

In eine Lösung von 19,1 g (0,03 Mol) 2-[4-(Benzolsulfonylthio)-3-tert.-butyloxycarbonylamido-2-oxo-azetidin-1-yl]-3-methylenbuttersäure-diphenylmethylester in 300 ml Essigester werden bei 0 bis -2° C innerhalb von 5 Min. 3,6 g (0,05 Mol) Chlorgas eingeleitet. Das Reaktionsgemisch wird 1,5 h bei 0° C nachgerührt, dann mit 300 ml gesättigter Natriumhydrogencarbonatlösung und 300 ml Wasser gewaschen. Die Essigesterphase wird mit Natriumsulfat getrocknet und im Vakuum eingedampft. Der Rückstand wird in 100 ml Isopropanol ausgerührt, der entstandene kristalline Niederschlag abgesaugt, mit Isopropanol gewaschen und im Vakuum getrocknet.
Ausbeute: 13,5 g Produkt = 67,1 % der Theorie.
NMR (250 MHZ, $CDCl_3$): 1,4[9]s, 4,05[1]d J = 14Hz, 4,15[1]d J = 14Hz, 4,55[1]s, 4,95[2]m, 5,15[1]s, 5,2-[1]s, 5,8[1]d J = 5Hz, 6,85[1]s, 7,25-7,55[13]m, 7,8[2]d J = 7Hz
$R_f$-Wert: 0,54 (Toluol/Essigester 8:2)

Beispiel 3

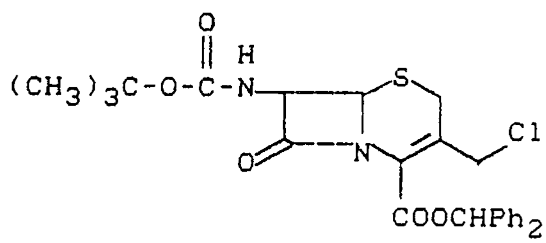

7-tert.-Butyloxycarbonylamido-3-chlormethyl-3-cephem-4-carbonsäure-diphenylmethylester

Zu einer Lösung von 0,63 g (0,037 Mol) Ammoniakgas in 35 ml DMF wird bei -40° C eine Lösung von 13,5 g (0,02 Mol) 2-[4-(Benzolsulfonylthio)-3-tert.-butyloxycarbonylamido-2-oxo-azetidin-1-yl]-4-chlor-3-methylenbuttersäure-diphenylmethylester in 50 ml DMF zugetropft. Die Reaktionsmischung wird 1 h bei -40° C bis -50° C nachgerührt, bei -40° C mit 14 ml 1n Salzsäure tropfenweise versetzt und mit 600 ml Wasser verdünnt. Der entstandene Niederschlag wird mit Wasser gewaschen und in 50 ml Methanol ausgerührt. Die Kristallsuspension wird abgesaugt, mit Methanol gewaschen und im Vakuum getrocknet.
Ausbeute: 9,5 g Produkt = 92 % der Theorie
NMR (300 MHZ, $CDCl_3$): 1,45[9]s, 3,5[1]d J = 16Hz, 3,65[1]d J = 16Hz, 4,35[1]d J = 13Hz, 4,45[1]d J = 13Hz, 5,0[1]d J = 5Hz, 5,25[1]d J = 10Hz, 5,65[1]dd J = 10Hz J = 5Hz, 6,95(1)s, 7,25-7,5[10]m
$R_f$-Wert: 0,72 (Toluol/Essigester 8:2)

Beispiel 4

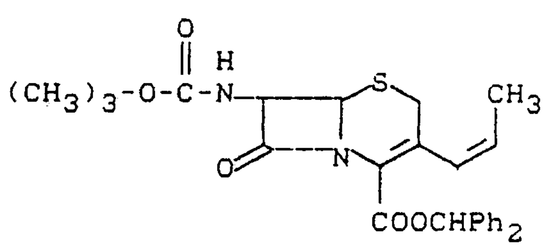

7-tert.-Butyloxycarbonylamido-3-(propen-1-yl)-3-cephem-4-carbonsäure-diphenylmethylester

2,57 g (5 mmol) 7-tert.-Butyloxycarbonylamido-3-chlormethyl-3-cephem-4-carbonsäure.diphenylmethylester werden in 20 ml Dichlormethan und 20 ml Isopropanol gelöst. Nach Zugabe von 0,84 g

(5 mmol) Kaliumjodid und 1,31 g (5 mmol) Triphenylphosphin wird 4 h bei 20 °C gerührt. Dann werden 4,4 g (100 mmol) Acetaldehyd und eine Lösung von 0,69 g (5 mmol) Kaliumcarbonat in 15 ml Wasser zugegeben und die Reaktionsmischung 30 Min. bei 20 °C kräftig gerührt. Die organische Phase wird abgetrennt, mit Magnesiumsulfat getrocknet und im Vakuum eingedampft. Der Rückstand wird durch Chromatographie an 300 g Kieselgel Si60 0,04 bis 0,063 mm mit Toluol-Essigester 9:1-Gemisch als Elutionsmittel gereinigt. Nach Eindampfen im Vakuum der geeigneten Fraktionen wird das Produkt als fester Schaum isoliert.
Ausbeute: 1 g Produkt (Gemisch der Z- und E-Isomere im Verhältnis 1:8) = 39,5 % der Theorie.
NMR (250 MHZ, DMSO-$d_6$): 1,35-1,4[12]m, 3,55(2)s, 5,2[1]d J = 5Hz, 4,9-5,1[2]m, 6,1[1]d J = 12Hz, 6,85-[1]s, 7,2-7,5[10]m, 8,0[1]d J = 10Hz
Die NMR-Daten gelten für das Z-Isomer.
$R_f$-Wert: 0,71 für das Z-Isomer (Toluol/Essigester 8:2)
0,73 für das E-Isomer (Toluol/Essigester 8:2).

Beispiel 5

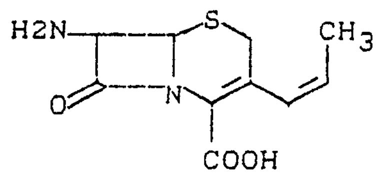

7-APCA

(7-Amino-3-(propen-1-yl)-3-cephem-4-carbonsäure)

5 g (10,85 mmol) 7-tert.-Butyloxycarbonylamido-3-(propen-1-yl)-3-cephemcarbonsäure-diphenylmethylester werden in 50 ml Trifluoressigsäure 3 h bei 20 °C gerührt. Dann werden unter Eiskühlung 300 ml Diisopropylether zugetropft und 15 Min. nachgerührt. Der ausgefallene Niederschlag wird abgesaugt, mit Diisopropylether gewaschen und in 50 ml Wasser suspendiert. Diese wäßrige Suspension wird mit ca. 3 ml 25 % Ammoniakwasser auf pH = 8,3 gebracht. Die wäßrige Suspension wird mit 2 g Aktivkohle versetzt und filtriert. Das Filtrat wird mit ca. 2 ml 6n Salzsäure auf pH = 2,5 gebracht. Der entstandene Niederschlag wird abgesaugt, mit Wasser und Aceton gewaschen und im Vakuum getrocknet.
Ausbeute: 2,0 g Produkt = 76,9 % der Theorie.
NMR (200 MHZ, DCOOD): 1,8[3]dd J = 7,5Hz J = 2Hz, 3,65[2]q J = 15Hz, 5,4[1]d J = 5Hz, 5,5[1]d J = 5Hz, 5,95[1]dq J = 12Hz J = 7,5Hz, 6,5[1]dd J = 12Hz J = 2Hz
Die NMR-Daten gelten für das Z-Isomer.
$R_f$-Wert: 0,5 für das Z-Isomer (n-Propanol-Essigester-Wasser 16:12:12)
0,52 für das E-Isomer (n-Propanol-Essigester-Wasser 16:12:12)

Beispiel 6

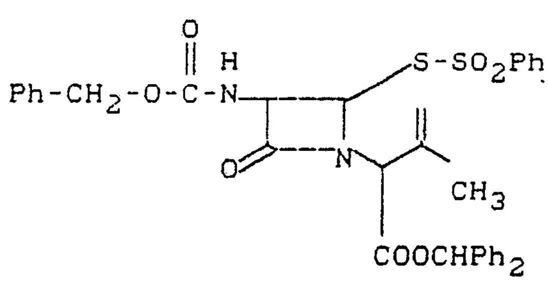

2-[4-(Benzolsulfonylthio)-3-benzyloxycarbonylamido-2-oxo-azetidin-2-yl]-3-methylenbuttersäure-diphenylmethylester

A. Synthese des Ausgangsmaterials

A.1.

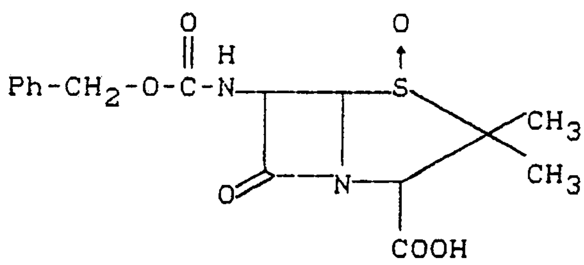

6-Benzyloxycarbonylamido-penicillansäure-1-oxid

108 g (0,5 Mol) 6-Aminopenicillansäure werden zusammen mit 185 g (2,2 Mol) Natriumhydrogencarbonat in 1500 ml Wasser gelöst. Dann wird bei 20° C unter kräftigem Rühren eine Lösung von 81 ml (0,54 Mol) Benzyloxycarbonylchlorid in 300 ml Diethylether innerhalb von 15 Min. zugetropft. Nach dem Ende des Zutropfens werden noch einmal 10 g (0,095 Mol) Natriumhydrogencarbonat in 250 ml Wasser gelöst zugegeben und 2 h bei 20° C nachgerührt. Dann wird dreimal mit je 300 ml Diethylether extrahiert, die wäßrige Phase mit 500 ml Essigester überschichtet und unter kräftigem Rühren mit 2n Schwefelsäure auf pH = 4 gebracht. Die wäßrige Phase wird noch zweimal mit je 1000 ml Essigester extrahiert, die vereinigten organischen Phasen mit Magnesiumsulfat getrocknet und im Vakuum eingedampft. Man erhält so 165,2 g festen, weißen Schaum, der zusammen mit 40,1 g (0,48 Mol) Natriumhydrogencarbonat in 2000 ml Wasser und 450 ml Ethanol gelöst wird. Bei 0° C werden dann 3,6 g (0,011 Mol) Natriumwolframat-Dihydrat zugegeben und 50 ml (0,49 Mol) 30 % Wasserstoffsuperoxid zugetropft. Die Reaktionsmischung wird 2 h bei 0° C nachgerührt, dann mit 2n Schwefelsäure auf pH = 3 gebracht und zweimal mit je 2000 ml Essigester extrahiert. Die vereinigten organischen Phasen werden mit Magnesiumsulfat getrocknet und im Vakuum zur Trockne eingedampft.
Ausbeute: 152,9 g Produkt = 83,5 % der Theorie.
NMR (250 MHZ, $CDCl_3$): 1,25[3]s, 1,7[3]s, 4,15[1]s, 5,0[1]d J = 5Hz, 5,15[2]s, 5,75[1]dd J = 10Hz J = 5Hz, 6,45[1]d J = 10Hz, 7,3[5]s, 7,65[1]s breit
$R_f$-Wert: 0,44 (Mobile Phase: Organische Phase einer kräftig geschüttelten Mischung von 300 ml Butylacetat, 36 ml n-Butanol, 100 ml Eisessig und 60 ml Pufferlösung pH 7).

A.2.

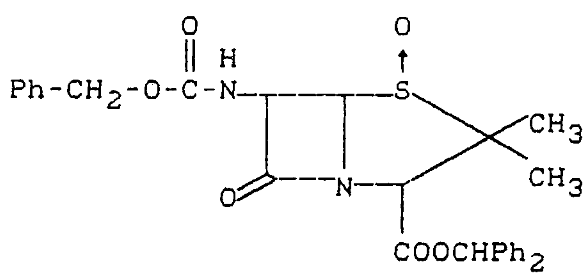

6-Benzyloxycarbonylamido-penicillansäure-diphenylmethylester-1-oxid

53,2 g (0,145 Mol) 6-Benzyloxycarbonylamido-penicillansäure-1-oxid werden in 200 ml Essigester gelöst. Bei 10° C werden zu dieser Lösung 250 ml einer 0,6 molaren Lösung von Diphenyldiazomethan in Toluol getropft. Die Reaktionslösung wird über Nacht bei 20° C gerührt und dann im Vakuum eingedampft.

Der ölige Rückstand wird an 1,2 kg Kieselgel Si60 0,04 bis 0,063 mm mit Toluol-Essigester 8:2-Gemisch als Elutionsmittel chromatographiert. Nach Eindampfen der geeigneten Fraktionen im Vakuum erhält man das Produkt als festen, weißen Schaum.
Ausbeute: 33,4 g Produkt = 43,2 % der Theorie
NMR (250 MHZ, $CDCl_3$): 1,35[3]s, 1,65[3]s, 4,75[1]s, 4,9[1]d J = 5Hz, 5,1[2]s, 5,75[1]dd J = 10Hz J = 5Hz, 6,4[1]d J = 10Hz, 6,95[1]s, 7,1-7,4[15]m
$R_f$-Wert: 0,55 (Toluol/Essigester 8:2)

A.3.

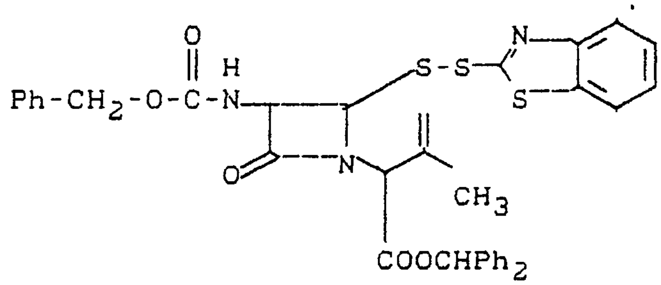

2-[4-(Benzthiazol-2-yldithio)-3-benzyloxycarbonylamido-2-oxo-azetidin-1-yl]-3-methylenbuttersäure-diphenylmethylester

39,8 g (74,7 mmol) 6-Benzyloxycarbonylamido-penicillansäure-diphenylmethylester-1-oxid werden zusammen mit 12,48 g (74,7 mmol) 2-Mercaptobenzothiazol in 450 ml Toluol am Wasserabscheider 1,5 h unter Rückfluß erhitzt. Dann wird die Reaktionslösung im Vakuum eingedampft und der Rückstand an 1500 g Kieselgel Si60 0,04bis 0,063 mm mit Toluol-Essigester 8:2-Gemisch als Elutionsmittel chromatographiert. Die geeigneten Fraktionen werden im Vakuum eingedampft und ergeben das Produkt als festen, weißen Schaum.
Ausbeute: 31,5 g Produkt = 62 % der Theorie.
NMR (250 MHZ, $CDCl_3$): 1,9[3]s, 4,95[1]s, 5,0-5,1[2]m, 5,15-5,25[2]m, 5,35[1]dd J = 10Hz J = 5Hz, 5,55-[1]d J = 5Hz, 6,35[1]d J = 10Hz, 6,9[1]s, 7,1-7,4[17]m, 7,65-7,8[2]m
$R_f$-Wert: 0,54 (Toluol/Essigester 8:2).

B.

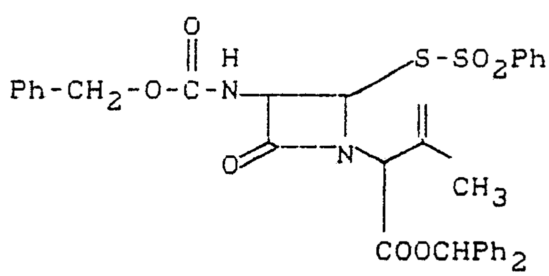

2-[4-(Benzolsulfonylthio)-3-benzyloxycarbonylamido-2-oxo-azetidin-2-yl]-3-methylenbuttersäure-diphenylmethylester

20,4 g (30 mmol) 2-[4-(Benzthiazol-2-yldithio)-3-benzyloxycarbonylamido-2-oxo-azetidin-1-yl]-3-methylenbuttersäure-diphenylmethylester werden in 80 ml Toluol bei 20°C nacheinander mit 10,1 g (60 mmol) Benzolsulfinsäure-Natriumsalz, 60 ml gesättigter Kochsalzlösung und 6 ml 6n Salzsäure versetzt. Die Reaktionsmischung wird 10 Min. kräftig gerührt. Der ausgefallene Niederschlag wird abgesaugt, im Filtrat wird die organische Phase abgetrennt. Zur organischen Phase werden dann 30 mg Kaliumjodid und 2,1 ml (21 mmol) 30 % Wasserstoffperoxid gegeben und 30 Min. bei 20°C gerührt. Dann wird mit Magnesiumsulfat getrocknet und im Vakuum eingedampft. Der ölige Rückstand wird an 700 g Kieselgel Si60 0,04 bis 0,063 mm mit Petrolether-Essigester 7:3-Gemisch als Elutionsmittel chromatographiert. Die geeigneten

Fraktionen werden im Vakuum eingedampft und ergeben das Produkt als festen, weißen Schaum.
Ausbeute: 16,5 g Produkt = 83,7 % der Theorie.
NMR (250 MHZ, $CDCl_3$): 1,75[3]s, 4,4[1]s, 4,8[1]s, 4,9[1]s, 5,0-5,15[3]m, 5,35[1]d J = 10Hz, 5,85[1]d J = 5Hz, 6,85[1]s, 7,2-7,55[18]m, 7,8[2]d J = 7Hz
$R_f$-Wert: 0,25 (Petrolether/Essigester 7:3)

Beispiel 7

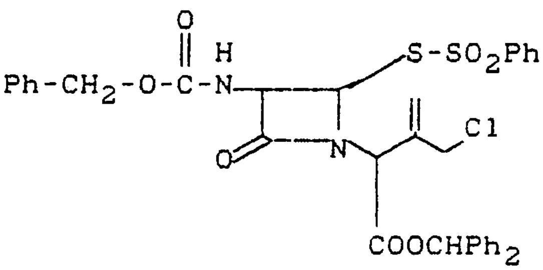

2-[4-(Benzolsulfonylthio)-3-benzyloxycarbonylamido-2-oxo-azetidin-1-yl]-4-chlor-3-methylenbuttersäure-diphenylmethylester

7,9 g (12 mmol) 2-[4-(Benzolsulfonylthio)-3-benzyloxycarbonylamido-2-oxo-azetidin-2-yl]-3-methylenbuttersäure-diphenylmethylester werden in 150 ml Essigester gelöst. Bei 0 bis 5° C werden dann 1,3 g (18 mmol) Chlorgas innerhalb von 10 Min. eingeleitet. Die Reaktionslösung wird 2 h bei 20° C nachgerührt und dann im Vakuum eingedampft. Der Rückstand wird an 100 g HD-SIL 18-30-60 0,02 bis 0,045 mm (Kronwald Separationstechnik GmbH) mit Acetonitril-Wasser 65:35-Gemisch als Elutionsmittel chromatographiert. Nach Eindampfen der geeigneten Fraktionen im Vakuum erhält man das Produkt als festen Schaum.
Ausbeute: 4,23 g Produkt = 51 % der Theorie.
NMR (250 MHZ, $CDCl_3$): 4,0[1]d J = 12Hz, 4,15[1]d J = 12Hz, 4,5[1]s, 4,9-5,10[4]m, 5,6[1]d J = 10Hz, 5,85[1]d J = 5Hz, 6,85[1]s, 7,2-7,45[18]m, 7,75[2]d J = 7Hz
$R_f$-Wert: 0,52 (Toluol/Essigester 8:2)

Beispiel 8

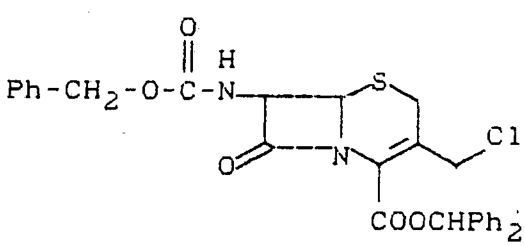

7-Benzyloxycarbonylamido-3-chlormethyl-3-cephem-4-carbonsäure-diphenylmethylester

Zu einer Lösung von 0,21 g (12,5 mmol) Ammoniakgas in 25 ml DMF wird bei -40° C eine Lösung von 3,75 g (5 mmol) 2-[4-(Benzolsulfonylthio)-3-benzyloxycarbonylamido-2-oxo-azetidin-1-yl]-4-chlor-3-methylenbuttersäure-diphenylmethylester in 16 ml DMF zugetropft. Nach 20 Min. Rühren bei -40° C werden 6 ml 1n Salzsäure bei -40° C zugetropft. Nach Verdünnen mit 300 ml Wasser wird der entstandene Niederschlag abgesaugt und mit Wasser gewaschen. Nach Trocknen im Vakuum wird der Niederschlag in

30 ml Diisopropylether ausgerührt, erneut abgesaugt und im Vakuum getrocknet.
Ausbeute: 1,9 g Produkt = 69 % der Theorie.
NMR (300 MHZ, $CDCl_3$): 3,45[1]d J = 16Hz, 3,6[1]d J = 16Hz, 4,35[1]d J = 12Hz, 4,45[1]d J = 12Hz, 4,95[1]d J = 5Hz, 5,15[2]s, 5,6[1]d J = 10Hz, 5,7[1]dd J = 10Hz J = 5Hz, 7,0[1]s, 7,2-7,5[15]m
$R_f$-Wert: 0,68 (Toluol/Essigester 8:2)

Beispiel 9

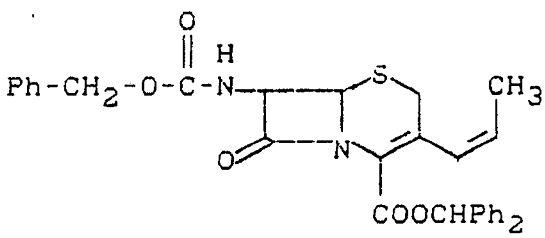

7-Benzylcxycarbonylamido-3-(propen-1-yl)-3-cephem-4-carbonsäure-diphenylmethylester

1,1 g (2 mmol) 7-Benzyloxycarbonylamido-3-chlormethyl-3-cephem-4-carbonsäure-diphenylmethylester werden in 10 ml Dichlormethan und 10 ml Isopropanol mit 0,33 g (2 mmol) Kaliumjodid und 0,52 g (2 mmol) Triphenylphosphin versetzt. Die Reaktionsmischung wird 4 h bei 20° C kräftig gerührt und dann mit 1,8 g (40 mmol) Acetaldehyd und 0,28 g (2 mmol) Kaliumcarbonat in 10 ml Wasser versetzt. Die Reaktionsmischung wird 30 Min. kräftig gerührt, dann werden die Phasen getrennt und die organische Phase mit Magnesiumsulfat getrocknet. Das nach Eindampfen im Vakuum erhaltene Öl wird an 120 g Kieselgel Si60 0,04 bis 0,063 mm mit Toluol-Essigester 9:1-Gemisch als Elutionsmittel chromatographiert. Nach Eindampfen der geeigneten Fraktionen im Vakuum wird das Produkt als fester Schaum erhalten.
Ausbeute: 0,45 g Produkt = 41 % der Theorie (Gemisch der E- und Z-Isomere im Verhältnis 1:9).
NMR (200 MHZ, DMSO-$d_6$): 1,4[3]d J = 7Hz, 3,55[2]s, 5,1[2]s, 5,2[1]d J = 5Hz, 5,4-5,65[2]s, 6,1[1]d J = 10Hz, 6,9[1]s, 7,2-7,55[15]m, 8,5[1]d J = 10Hz
Die NMR-Daten gelten für das Z-Isomer.
$R_f$-Wert: 0,63 für das Z-Isomer (Toluol/Essigester 8:2)
0,69 für das E-Isomer (Toluol/Essigester 8:2).

Beispiel 10

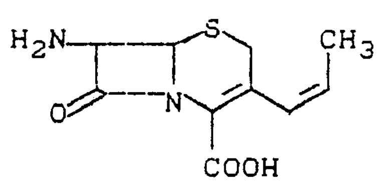

7-APCA

(7-Amino-3-(propen-1-yl)-3-cephem-4-carbonsäure)

Methode A:

0,55 g (1 mmol) 7-Benzyloxycarbonylamido-3-(propen-1-yl)-3-cephem-4-carbonsäure-diphenylmethylester werden in 5 ml Dichlormethan unter Stickstoff mit 0,8 g (4 mmol) Trimethylsilyljodid versetzt und bei 20°C 2 h gerührt. Dann wird die Reaktionslösung mit 50 ml 2n Schwefelsäure extrahiert und die wäßrige Phase mit 25 % wäßrigem Ammoniak auf pH = 3,5 gebracht. Der ausgefallene Niederschlag wird abgesaugt, mit Wasser und Aceton gewaschen und im Vakuum getrocknet.
Ausbeute: 0,13 g Produkt = 55 % der Theorie.

Methode B:

1,1 g (2 mmol) 7-Benzyloxycarbonylamido-3-(propen-1-yl)-3-cephem-4-carbonsäure-diphenylmethylester werden in 10 ml Trifluoressigsäure gelöst und mit 0,93 g (8 mmol) Triethylsilan versetzt. Nach 2h Rühren bei 20°C werden unter Eiskühlung 60 ml Diisopropylether zugetropft und 20 Minuten nachgerührt. Der ausgefallene Niederschlag wird abgesaugt, mit Diisopropylether gewaschen und in 20 ml Wasser suspendiert. Diese wäßrige Suspension wird mit ca. 1 ml 25 % Ammoniakwasser auf pH = 8,3 gebracht. Die wäßrige Suspension wird mit 1 g Aktivkohle versetzt und filtriert. Das Filtrat wird mit ca. 0,5 ml 6n Salzsäure auf pH = 2,5 gebracht. Der entstandene Niederschlag wird abgesaugt, mit Wasser und Aceton gewaschen und im Vakuum getrocknet.
Ausbeute: 0,091 g Produkt = 19 % der Theorie.

Methode C:

Zu einer Lösung von 5 g (40 mmol) Bortribromid in 40 ml Dichlormethan werden bei 0°C 13,7 g = 9,25 ml (120 mmol) Trifluoressigsäure zugetropft. Die resultierende Suspension wird im Vakuum zur Trockne eingedampft. Der Rückstand wird in 40 ml Trifluoressigsäure gelöst und bei -20°C zu einer Lösung von 2,75 g (5 mmol) 7-Benzyloxycarbonylamido-3-(propen-1-yl)-3-cephem-4-carbonsäure-diphenylmethylester und 2 ml Anisol gegeben. Die Reaktionsmischung wird 1h bei 0°C gerührt, dann im Vakuum zur Trockne eingedampft. Der Rückstand wird aus Diethylether kristallisiert, abgesaugt und in 40 ml Wasser suspendiert. Die wäßrige Suspension wird mit konz. Salzsäure auf pH = 0,3 gebracht, 5 Min. gerührt und dann filtriert. Das Filtrat wird unter Eiskühlung mit 25 % wäßrigem Ammoniak auf pH = 3,0 gebracht. Der ausgefallene Niederschlag wird abgesaugt, mit Wasser gewaschen und im Vakuum getrocknet.
Ausbeute: 0,87 g Produkt = 72,5 % der Theorie.

Beispiel 11

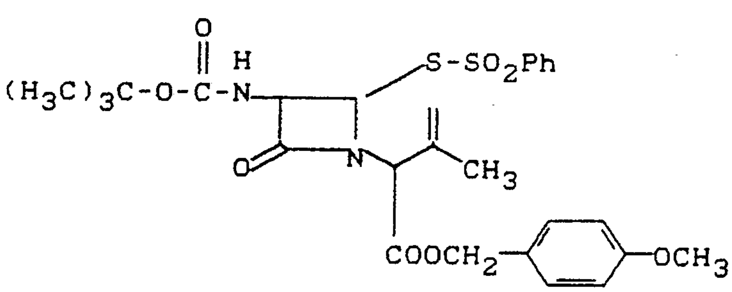

2-[4-(Benzolsulfonylthio)-3-tert.-butyloxycarbonylamido-2-oxo-azetidin-1-yl]-3-methylenbuttersäure-(4-methoxybenzyl)ester

A. Synthese des Ausgangsmaterials

A.1.

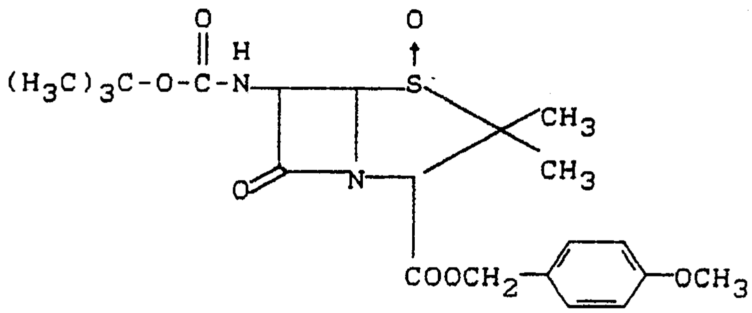

6-tert.-Butyloxycarbonylamido-penicillansäure-(4-methoxybenzyl)ester

132,9 g (0,4 Mol) 6-tert.-Butyloxycarbonylamido-penicillansäure-1-oxid werden in 400 ml DMF gelöst. Dann werdem 54 ml (0,4 Mol) Triethylamin und 93,9 g (0,6 Mol) 4-Methoxybenzylchlorid bei 5-10 °C zugegeben. Nach Zugabe von 66,4 g (0,4 Mol) Kaliumjodid wird die Reaktionsmischung 16h bei 25 °C gerührt. Dann werden 1500 ml Wasser unter kräftigem Rühren in die Reaktionsmischung getropft und 3h nachgerührt. Der entstandene Niederschlag wird abgesaugt, in 1000 ml Aceton bei 35 °C gelöst und bei 20 °C langsam mit 1000 ml Wasser versetzt. Es wird 1h bei 20 °C nachgerührt, dann wird der weiße, kristalline Niederschlag abgesaugt, mit 500 ml Wasser und 500 ml Wasser-Aceton im Verhältnis 1:1 gewaschen und im Vakuum getrocknet.
Ausbeute: 136,2 g Produkt = 75,7 % der Theorie
NMR (250 MHZ, $CDCl_3$): 1,05[3]s, 1,45[9]s, 165[3]s, 3,8[3]s, 4,65[1]s, 5,0[1]d J = 5Hz, 5,1[1]d J = 12Hz, 5,25[1]d J = 12Hz, 5,7[1]dd J = 10Hz J = 5Hz, 6,05[1]d J = 10Hz, 6,9[2]d J = 8Hz, 7,3[2]d J = 8Hz
$R_f$-Wert: 0,33 (Toluol/Essigester 8:2).

A.2

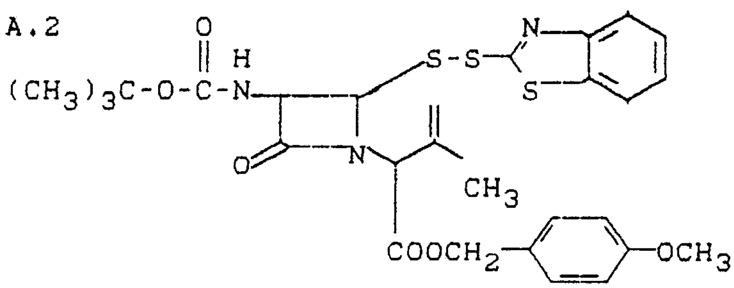

2-[4-(Benzthiazol-2-yldithio)-3-tert.-butyloxycarbonylamido-2-oxo-azetidin-1-yl]-3-methylenbuttersäure-(4-methoxybenzyl)ester

90,4 g (0,2 Mol) 6-tert.-Butyloxycarbonylamido-penicillansäure-(4-methoxybenzyl)ester werden zusammen mit 34,3 g (0,2 Mol) 2-Mercaptobenzothiazol in 1600 ml Toluol 1h am Wasserabscheider unter Rückfluß erhitzt. Anschließend wird die Reaktionslösung im Vakuum zur Trockne eingedampft. Der ölige Rückstand wird in 500 ml Diisopropylether 16h verrührt. Der entstandene kristalline Niederschlag wird abgesaugt und im Vakuum getrocknet.
Ausbeute: 77,4 g = 64,2 % der Theorie
NMR (300 MHZ, $CDCl_3$): 1,5[9]s, 1,9[3]s, 3,8[3]s, 4,95[1]s, 5,0[1]s 5,15[2] AB-Quartett, 5,2[1]s, 5,35[1]dd J = 10Hz, J = 5Hz, 5,5[1]d J = 5Hz, 5,65[1]d J = 10Hz, 6,85[2]d J = 8Hz, 7,25[2]d J = 8Hz, 7,35[1]tr J = 8Hz, 7,45[1]tr J = 8Hz, 7,8[1]d J = 8Hz, 7,9[1]d J = 8Hz
$R_f$-Wert: 0,43 (Toluol/Essigester 8:2)

B.

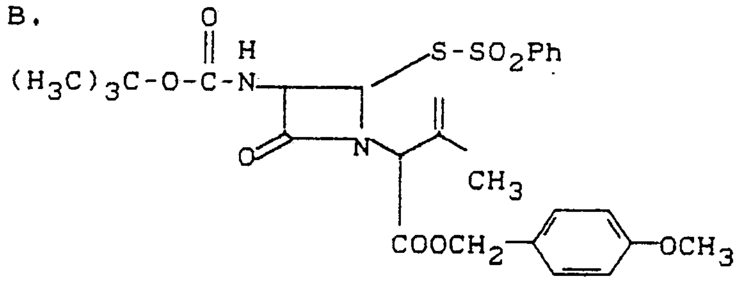

2-[4-(Benzolsulfonylthio)-3-tert.-butyloxycarbonylamido-2-oxo-azetidin-1-yl]-3-methylenbuttersäure-(4-methoxybenzyl)ester

30 g (0,05 Mol) 2-[4-(Benzthiazol-2-yldithio)-3-tert.-butyloxycarbonylamido-2-oxo-azetidin-1-yl]-3-methylen buttersäure-(4-methoxybenzyl)ester werden in 100 ml Toluol gelöst und mit 100 ml gesättigter Kochsalzlösung und 16,4 g (0,1 Mol) Benzolsulfinsäure-Natriumsalz versetzt. Nach Zutropfen von 17 ml 6n Salzsäure wird die Reaktionsmischung 3h bei 20 °C gerührt. Der entstandene Niederschlag wird abgesaugt, im Filtrat werden die Phasen getrennt und die organische Phase wird mit 6 ml 30 %igem Wasserstoffsuperoxid versetzt und 16h bei 20 °C gerührt. Nach Trocknen mit Natriumsulfat wird die Reaktionslösung im Vakuum eingedampft, das resultierende Öl wird in 200 ml Diisopropylether 3h verrührt. Der entstandene Niederschlag wird abgesaugt und mit Diisopropylether gewaschen. Der Niederschlag wird in 200 ml Diethylether ausgerührt, abgesaugt, mit wenig Diethylether gewaschen und im Vakuum getrocknet.

Ausbeute: 14 g Produkt = 48,6 % der Theorie

NMR (250 MHZ, $CDCl_3$): 1,4[9]s, 1,75[3]s, 3,85[3]s, 4,55[1]s, 4,75[1]s, 4,85[1]s 5,0-5,2[4]m, 5,75[1]d J = 5Hz, 6,9[2]d J = 8Hz, 7,3[2]d J = 8Hz, 7,45-7,65[3]m, 7,85[2]d J = 8Hz

$R_f$-Wert: 0,32 (Toluol/Essigester 8:2)

Beispiel 12

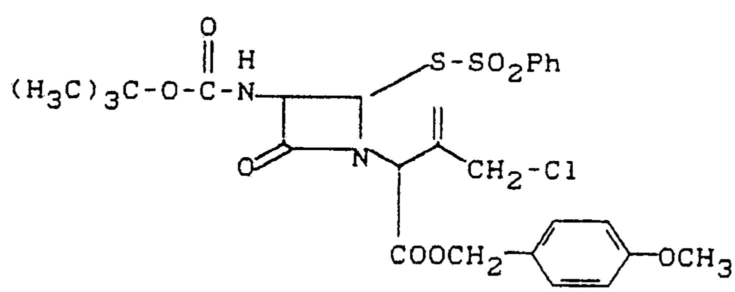

2-[4-(Benzolsulfonylthio)-3-tert.-butyloxycarbonylamido-2-oxo-azetidin-1-yl]-4-chlor-3-methylenbuttersäure-(4-methoxybenzyl)ester

22 g (0,038 Mol) 2-[4-(Benzolsulfonylthio)-3-tert.-butyloxycarbonylamido-2-oxo-azetidin-1-yl]-3-methylenbuttersäure-(4-methoxybenzyl)ester werden in 120 ml Essigester gelöst und mit 120 ml Wasser versetzt. Bei 0-5 °C werden dann 4 g (0,056 Mol) Chlorgas innerhalb von 10 Min. eingeleitet. Es wird 30 Min. bei 0 °C nachgerührt, dann werden 8,4 g (0,1 Mol) Natriumhydrogencarbonat und 12,4 g (0,05 Mol) Natriumthiosulfat-Pentahydrat zugegeben. Nach 10-minütigem Rühren wird die Essigesterphase abgetrennt, mit Magnesiumsulfat getrocknet und im Vakuum eingedampft. Der Rückstand wird an 300 g HD-SIL 18-30-60 0,02 bis 0,045 mm (Kronwald Separationstechnik GmbH) mit Acetonitril-Wasser 65:35-Gemisch als Elutionsmittel chromatographiert. Nach Eindampfen der geeigneten Fraktionen im Vakuum erhält man das Produkt als festen Schaum.

Ausbeute: 15,3 g Produkt = 66 % der Theorie

NMR (250 MHZ, $CDCl_3$): 1,4[9]s, 3,8[3]s, 4,1[2]q J = 12Hz, 4,75[1]s, 4,95-5,15[5]m, 5,2[1]s, 5,8[1]d J = 5Hz, 6,9[2]d J = 8Hz, 7,3[2]d J = 8Hz, 7,5[2]tr J = 8Hz, 7,6[1]tr J = 8Hz, 7,85[2]d J = 8Hz
$R_f$-Wert: 0,33 (Toluol/Essigester 8:2)

Beispiel 13

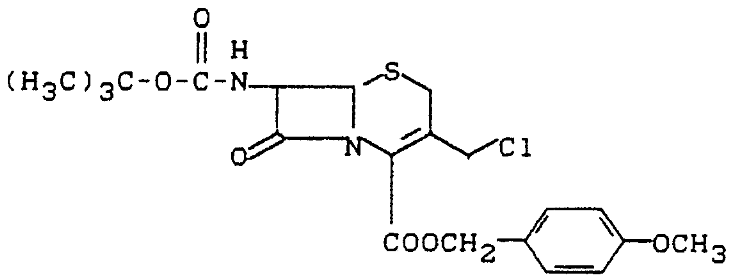

7-tert.-Butyloxycarbonylamido-3-chlormethyl-3-cephem-4-carbonsäure-(4-methoxybenzyl)ester

15,3 g (0,025 Mol) 2-[4-(Benzolsulfonylthio)-3-tert.-butyloxycarbonylamido-2-oxo-azetidin-1-yl]-4-chlor-3-methylenbuttersäure-(4-methoxybenzyl)ester werden in 50 ml DMF gelöst und bei -40° C zu einer Lösung von 4,2 ml (0,056 Mol) 25 %igem Ammoniakwasser in 20 ml DMF getropft. Es wird 30 Min. bei -40° C nachgerührt, dann werden 20 ml 1n Salzsäure bei -40° C zugetropft. Das Reaktionsgemisch wird unter Rühren in 500 ml Wasser gegossen und 1h gerührt. Der entstandene Niederschlag wird abgesaugt, in Essigester gelöst und dreimal mit Wasser gewaschen.

Die Essigesterlösung wird mit Magnesiumsulfat getrocknet und im Vakuum eingedampft. Der Rückstand wird an 240 g Kieselgel Si60 0,04 bis 0,063 mm mit Toluol/Essigester 95:5-Gemisch als Elutionsmittel chromatographiert. Nach Eindampfen der geeigneten Fraktionen im Vakuum erhält man das Produkt als festen Schaum.

Ausbeute: 9,6 g Produkt = 82 % der Theorie
NMR (250 MHZ, $CDCl_3$): 1,45[9]s, 3,5[1]d, J = 18Hz, 3,65[1]d J = 18Hz, 3,8[3]s, 4,4[1]d J = 12Hz, 4,55-[1]d J = 12Hz, 4,95[1]d J = 5Hz, 5,2[1]d J = 10Hz, 5,25[2]s, 5,6[1]dd J = 10 Hz, J = 5Hz, 6,9[2]d J = 8Hz, 7,3[2]d J = 8 Hz
$R_f$-Wert: 0,65 (Toluol/Essigester 8:2)

Beispiel 14

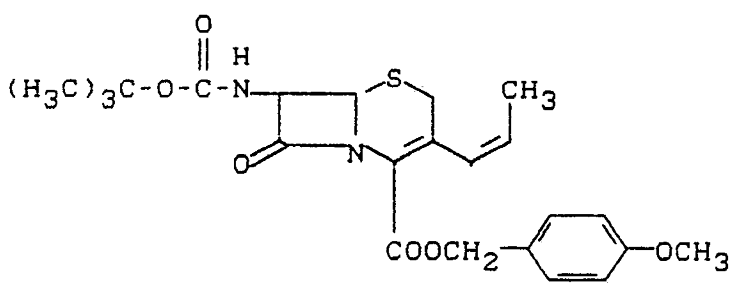

7-tert.-Butyloxycarbonylamido-3-(propen-1-yl)-3-cephem-4-carbonsäure-(4-methoxybenzyl)ester

9,3 g (0,02 Mol) 7-tert.-Butyloxycarbonylamido-3-chlormethyl-3-cephem-4-carbonsäure-(4-methoxybenzyl)ester werden in 100 ml Dichlormethan gelöst. Nach Zugabe von 100 ml Isopropanol, 4,6 g (0,02 Mol) Triphenylphosphin und 3,3 g (0,02 Mol) Kaliumjodid wird die Reaktionsmischung 4h bei 20° C kräftig

gerührt. Dann werden 22,3 ml (0,4 Mol) Acetaldehyd und eine Lösung von 2,76 g (0,02 Mol) Kaliumcarbonat in 100 ml Wasser zugegeben. Die Re aktionsmischung wird 1h kräftig gerührt, dann werden die Phasen getrennt und die org. Phase mit Magnesiumsulfat getrocknet. Die org. Phase wird im Vakuum eingedampft, der Rückstand kristallisiert aus Isopropanol. Der entstandene Niederschlag wird abgesaugt, mit Isopropanol gewaschen und im Vakuum getrocknet.
Ausbeute: 3,0 g Produkt (Gemisch der Z- und E-Isomere im Verhältnis 1:8) = 33 % der Theorie
NMR (200 MHZ, DMSO-$d_6$): 1,4[9]s, 1,5[3]dd J = 7Hz J = 2Hz, 3,55[2]AB-System, 3,8[3]s, 5,05[1]d J = 12 Hz, 5,1[1]d J = 5Hz, 5,15[1]d J = 12Hz, 5,4[1]dd J = 8Hz J = 5Hz, 5,6[1]dq J = 10Hz J = 7Hz, 6,1-[1]d J = 10Hz, 6,9[2]d J = 8Hz, 7,3[2]d J = 8Hz, 8,0[1]d J = 8H
Die NMR-Daten gelten für das Z-Isomer.
$R_f$-Wert: 0,64 für das Z-Isomer (Toluol/Essigester 8:2) 0,67 für das E-Isomer (Toluol/Essigester 8:2)

Beispiel 15

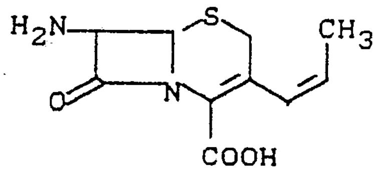

7-APCA

(7-Amino-3-(propen-1-yl)-3-cephem-4-carbonsäure)

2,76 g (6 mmol) 7-tert.-Butyloxycarbonylamido-3-(propen-1-yl)-3-cephem-4-carbonsäure-(4-methoxybenzyl)ester werden in 25 ml Trifluoressigsäure 3h bei 20°C gerührt. Dann werden unter Eiskühlung 300 ml Diisopropylether zugetropft und 15 Min. nachgerührt. Der angefallene Niederschlag wird abgesaugt, mit Diisopropylether gewaschen und in 25 ml Wasser suspendiert. Diese wäßrige Suspension wird mit etwa 1,5 ml 25 %igem Ammoniakwasser auf pH = 8,3 gebracht. Die wäßrige Suspension wird mit 1 g Aktivkohle versetzt und filtriert. Das Filtrat wird mit ca. 1 ml konz. Salzsäure auf pH = 2,5 gebracht. Der entstandene Niederschlag wird abgesaugt, mit Wasser gewaschen und im Vakuum getrocknet.
Ausbeute: 1,0 g Produkt = 69,4 % der Theorie.

**Ansprüche**

1. Verfahren zur Herstellung der Verbindung 7-Amino-3-[(Z)-1-propen-1-yl]-3-cephem-4-carbonsäure (7-APCA) (I), dadurch gekennzeichnet, daß man Verbindungen der Formel (II),

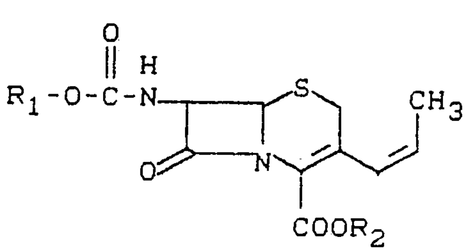

(II)

in der
$R_1$ und $R_2$ gleich oder verschieden sein können und für Alkyl-, Aryl- und Aralkylreste stehen, durch Behandlung mit starken Protonsäuren oder mit Lewissäuren in die Verbindung

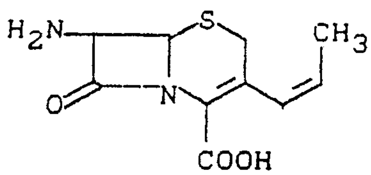

(I)

überführt.

2. Verfahren zur Herstellung von Verbindungen der Formel (II)

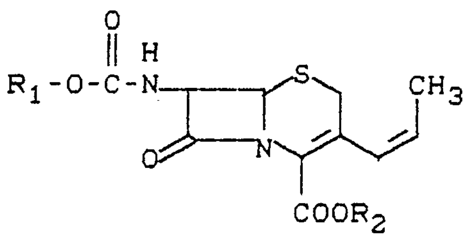

(II)

in der $R_1$ und $R_2$ die in Anspruch 1 angegebene Bedeutung haben, dadurch gekennzeichnet, daß man zu einer Verbindung der Formel (VI)

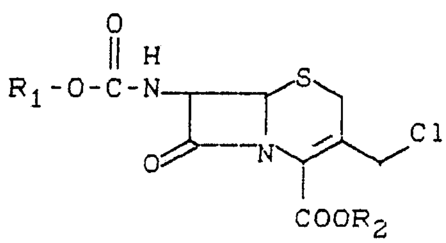

(VI)

in der $R_1$ und $R_2$ die in Anspruch 1 angegebene Bedeutung haben, 1 bis 2 Äquivalente Triphenylphosphin und 0,1 bis 2 Äquivalente Alkalijodid zusetzt, anschließend mit 3 bis 50 Äquivalenten Acetaldehyd umsetzt und mit einer Base behandelt.

3. Verfahren zur Herstellung von Verbindungen der allgemeinen Formel (VI)

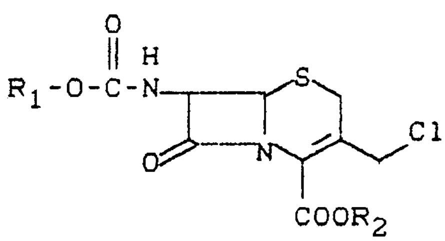

(VI)

in der $R_1$ und $R_2$ die in Anspruch 1 angegebene Bedeutung haben, dadurch gekennzeichnet, daß man Verbindungen der Formel (VII)

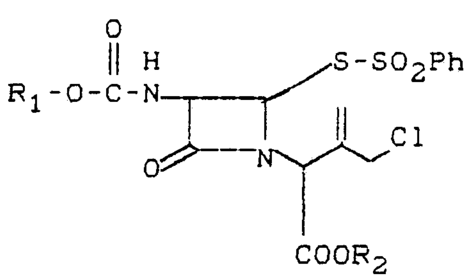

(VII)

in der $R_1$ und $R_2$ ebenfalls die in Anspruch 1 angegebene Bedeutung haben, in einem polaren Lösungsmittel bei Temperaturen von -70° C bis 0° C mit 1 bis 4 Äquivalenten einer Base behandelt.

4. Verfahren zur Herstellung der Verbindungen der Formel (VII)

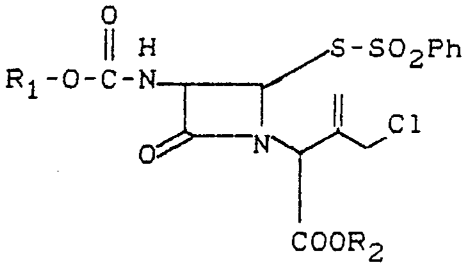

(VII)

in der $R_1$ und $R_2$ die in Anspruch 1 angegebene Bedeutung haben, dadurch gekennzeichnet, daß man Verbindungen der Formel (VIII)

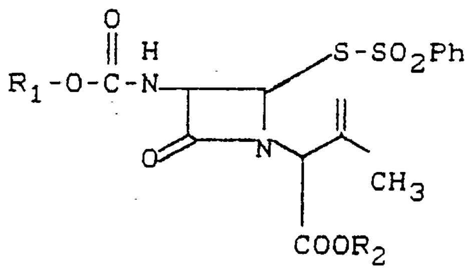

(VIII)

in der $R_1$ und $R_2$ die in Anspruch 1 angegebene Bedeutung haben, in einem organischen Lösungsmittel mit einem Chlorierungsmittel behandelt.